# EUROPEAN PATENT APPLICATION

(11) **EP 4 289 447 A1**
(43) Date of publication of application: **13.12.2023**
(21) Application number: 23177396.1
(22) Date of filing: 05.06.2023
(51) Int. Cl.: A61L 2/10, A61L 2/24, E03D 9/00

(54) **PROXIMITY BASED ULTRAVIOLET DOSAGE CONTROL**

(30) Priority: 08.06.2022 US 202217835785
(71) Applicant: B/E Aerospace, Inc., Winston-Salem, NC 27105 (US)
(72) Inventor: EDQUIST, John, Milwaukee, WI, 53207 (US); JOHANNESSEN, Eric, Holbrook, NY, 11741 (US)
(74) Representative: Dehns

(57) **Abstract**

A of using an ultraviolet (UV) light emitter is disclosed herein. The method includes measuring, by a processor (310), a first distance from the UV light emitter (302) to a target surface, activating, by the processor, the UV light emitter, and recording, by the processor, a second distance from the UV light emitter to the target surface. The method further includes calculating, by the processor, an accumulated dose of UV light on the target surface based at least in part on the second distance and deactivating, by the processor, the UV light emitter in response to the accumulated dose reaching a target dose.

## Description

### FIELD

The present disclosure generally relates ultraviolet lighting in lavatories, and more specifically, to controlling ultraviolet lighting in lavatories.

### BACKGROUND

Ultraviolet (UV) lighting technologies perform germicidal function on surfaces but can also have impacts to human health and material degradation. Assuming constant emission of UV energy, total dose received on a surface varies by distance from the UV source and receiving surface. Typically, a UV emitter seeks to apply a consistent dose of energy which inactivates a target percentage of pathogens (i.e., virus). There is no benefit to applying extra dose beyond the target amount, but it may create a hazard for materials. For a fixed installation of an overhead UV emitter, the distance to the target changes depending on the ceiling height or other objects placed directly below the emitter. This variability complicates controlling UV dosage.

### SUMMARY

A of using an ultraviolet (UV) light emitter is disclosed herein. The method includes measuring, by a processor, a first distance from the UV light emitter to a target surface, activating, by the processor, the UV light emitter, and recording, by the processor, a second distance from the UV light emitter to the target surface. The method further includes calculating, by the processor, an accumulated dose of UV light on the target surface based at least in part on the second distance and deactivating, by the processor, the UV light emitter in response to the accumulated dose reaching a target dose.

In various embodiments, the recording the second distance occurs once every second. In various embodiments, the method further includes recording, by the processor, an accumulated time since activating the UV light emitter and calculating, by the processor, the accumulated dose of UV light based at least in part on the accumulated time and the recorded second distance. In various embodiments, the calculating the accumulated dose includes integrating the second distance by an energy output of the UV light emitter over an accumulated time period.

In various embodiments, the method further includes measuring, by the processor, a third distance from the UV light emitter and detecting, by the processor, a movement in response to the third distance being different than the second distance. In various embodiments, the method further includes changing, by the processor, the target dose to a lower dose in response to the third distance being less than the second distance. In various embodiments, the method further includes measuring, by the processor, a fourth distance from the UV light emitter and maintaining, by the processor, the target dose in response to the fourth distance being similar to the second distance. In various embodiments, the method further includes measuring, by the processor, a time between the second distance measurement and the fourth distance measurement and determining, by the processor, that the time is below a threshold.

In various embodiments, the method further includes repeating, by the processor, the recording and the calculating until the target dose reached. In various embodiments, the measuring the first distance is performed in response to a trigger.

Also disclosed herein is a system for using an ultraviolet (UV) light emitter in an aircraft lavatory. The system includes a UV light source, an occupancy sensor, and a UV light source driver. The UV light source driver includes a power source configured to power the UV light source, a processor, a memory coupled to the processor. The memory comprising instructions when executed by the processor cause the processor measure a first distance from the occupancy sensor to a target surface, activate the UV light source, record a second distance from the occupancy sensor to the target surface, calculate an accumulated dose of UV light on the target surface based at least in part on the second distance, and deactivate the UV light source in response to the accumulated dose reaching a target dose.

In various embodiments, the instructions when executed by the processor further cause the processor to record the second distance each time period and calculate the accumulated dose each time period. In various embodiments, the instructions when executed by the processor further cause the processor to record an accumulated time since activating the UV light source and calculate the accumulated dose based at least in part on the accumulated time and the recorded second distance. In various embodiments, the calculating the accumulated dose includes integrating the second distance by an energy output of the UV light emitter over an accumulated time period.

In various embodiments, the instructions when executed by the processor further cause the processor to measure a third distance from the occupancy sensor to the target surface and detect a movement in response to the third distance being different than the second distance. In various embodiments, the instructions when executed by the processor further cause the processor to change the target dose to a lower dose in response to the third distance being less than the second distance.

In various embodiments, the instructions when executed by the processor further cause the processor to measure a fourth distance from the occupancy sensor and determine to maintain the target dose in response to the fourth distance being similar to the second distance. In various embodiments, the instructions when executed by the processor further cause the processor to measure a time between the second distance measurement and the fourth distance measurement and determine that the time is below a threshold before determining to maintain the target dose.

In various embodiments, the instructions when executed by the processor further cause the processor to begin the measuring the first distance in response to a trigger. In various embodiments, the instructions when executed by the processor further cause the processor to repeat the recording and the calculating until the target dose is reached.

The foregoing features and elements may be combined in any combination, without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1 illustrates a lavatory including an ultraviolet lighting module, in accordance with various embodiments.
FIGS. 2A and 2B illustrate an ultraviolet lighting module, in accordance with various embodiments.
FIG. 3 illustrates a system schematic for controlling an ultraviolet lighting module, in accordance with various embodiments.
FIG. 4 illustrates a flow diagram of a method for controlling an ultraviolet lighting module, in accordance with various embodiments.
FIGS. 5A and 5B illustrate ultraviolet lighting dosage graphs, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the invention as defined by the claims. Thus, the detailed description herein is presented for purposes of illustration only and not of limitation. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

Disclosed herein is a system for applying a dose of ultraviolet (UV) energy to a surface by a UV emitter. The UV emitter may be used to sanitize surfaces in an aircraft lavatory, though other uses and locations are contemplated. A dose of UV energy may be measured as power (e.g., watts) per unit area (e.g., cm²) over a period of time (e.g., seconds) resulting in an accumulated energy (e.g., joules) per unit area (e.g., cm²). Accordingly, doses will be described below as the resulting accumulated dose (e.g., mJ/cm²) that are the result of a given amount of UV energy (e.g., watts) over a period of time (e.g., seconds). When using UV energy for sanitization, the calculation of an amount of energy reaching target surface correlates to a reduction of germs on the surface. For example, a 222 nm UV light kills about 90% of SARS-CoV-2 virus on a surface once a 1 mJ/cm² dose is reached. As another example, a 222 nm UV light kills about 99.9% of COVID-19 virus on a surface once a 3 mJ/cm² dose is reached. As more energy is accumulated (e.g., total dose) on the surface, a larger percentage of a given pathogen is killed.

The total accumulated dose of UV energy is dependent, at least in part, on the distance from the UV emitter to the receiving surface, the strength of the UV emitter, and the duration of exposure. For example, a longer exposure duration may be used for a surface that is further away from the UV emitter than for a surface that is closer to the UV emitter. An active infrared (IR) proximity sensor may be placed in the centerline of the UV emitter and can actively measure the distance between the UV emitter and receiving surface many times per second. The UV emitter may include an internal processor that can use this distance to calculate appropriate ON time duration to reach a target dose. By using active proximity, the sensor may also be used to detect movement. If the distance changes during an ON cycle, the processor may actively measure the accumulated dose and turn OFF the UV emitter. This may also lower the risk that human exposure levels exceed safe limits defined by regulatory requirements. This may also minimize overexposure of surfaces to limit material discoloration and degradation. Furthermore, this allows a universal solution to be offered throughout the aircraft and across aircrafts without requiring a unique software solution to control the dose or UV emitter ON time duration in each installation

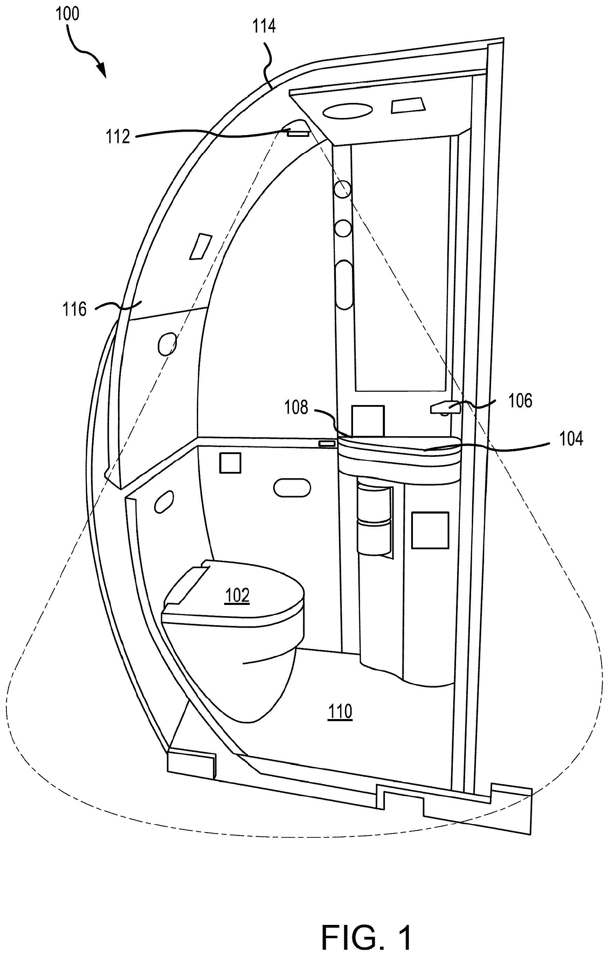

Referring now to FIG. 1, in accordance with various embodiments, an aircraft lavatory 100 is illustrated. Aircraft lavatory 100 includes a toilet 102, a sink 104, a faucet 106, a counter 108, a floor 110, and an ultraviolet (UV) light emitter 112, also referred to as UV emitter 112. UV emitter 112 may be placed in a ceiling 114 of aircraft lavatory 100 or a wall 116 of aircraft lavatory 100. As illustrated, UV emitter 112 is placed in the ceiling 114 of aircraft lavatory 100 and is able to emit UV energy onto toilet 102, sink 104, faucet 106, counter 108, and floor 110, among other fixtures in aircraft lavatory 100.

As can be seen in FIG. 1, each of floor 110, toilet 102, counter 108, and ceiling 114 are at different distances from UV emitter 112. For example, floor 110 may be about 72 inches (182.9 cm) from UV emitter 112, toilet 102 may be about 48 inches (121.9 cm) from UV emitter 112, counter 108 may be about 36 inches (91.5 cm) from UV emitter 112, and ceiling 114 may be flush with, or about 0 inches (0 cm) from, UV emitter 112. The head of a passenger entering aircraft lavatory 100 may be between about 0 inches (0 cm) and about 36 inches (91.5 cm) from UV emitter 112. Each of these distances are exemplary and for discussion purposes of this disclosure. It should be appreciated that the distances may vary depending on the type of aircraft, the type of lavatory, the fixtures within the lavatory, and the height of the passenger, among other variables.

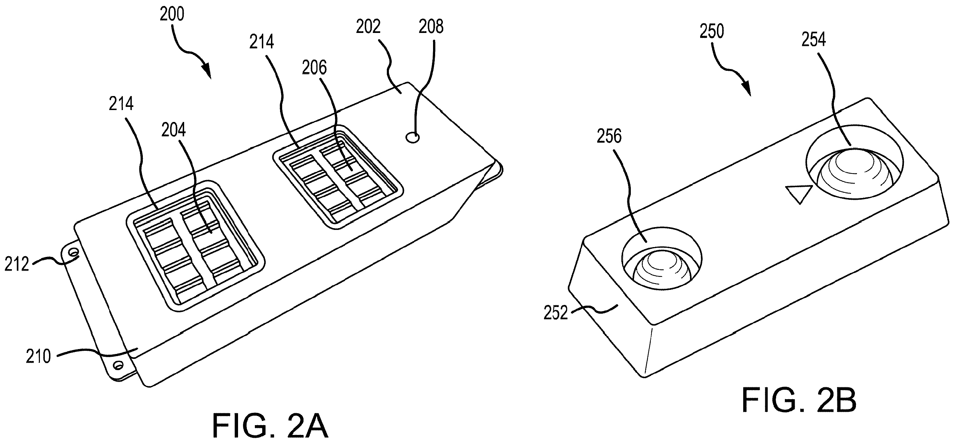

Referring now to FIGS. 2A and 2B, in accordance with various embodiments, a UV emitter 200 including an occupancy sensor 250 are illustrated. UV emitter 200 may be an example of UV emitter 112 described above with respect to FIG. 1. UV emitter 200 includes a housing 202 that holds a first UV light source 204 and a second UV light source 206. Housing 202 further includes a hole 208 through which occupancy sensor 250 operates. Housing 202 may further include a power supply to power first and second UV light sources 204, 206 and additional electronics and components for operating UV emitter 200. Housing 202 may be made from metal, plastic, or a composite material, among others. In various embodiments, housing 202 is mounted to ceiling 114. In various embodiments, housing 202 is mounted in ceiling 114 such that a surface 210 of housing 202 is visible. Housing 202 may include fastener holes 212 to receive fasteners for mounting housing 202 to, or in, ceiling 114.

First and second UV light sources 204, 206 may emit UV light having a wavelength of about 180 nm to about 300 nm, and more specifically, from about 200 nm to about 265 nm. The wavelength of first and second UV light sources 204, 206 may determine the radiant power UV emitter 200. First and second UV light sources 204, 206 further include reflectors 214 for reflecting the UV light away from housing 202. Variations in reflectors 214 may further affect the radiant power of UV emitter 200.

Occupancy sensor 250 is mounted inside housing 202 of UV emitter 200. Occupancy sensor 250 includes a housing 252, an infrared (IR) emitter 254, and an IR sensor 256. Housing 252 may further include additional electronics and components for operating occupancy sensor 250. Occupancy sensor 250 uses IR emitter 254 and IR sensor 256 to detect the proximity, or distance, to an object (e.g., toilet 102, counter 108, floor 110, etc.). To determine the distance to the object, IR transmitter 254 transmits a pulse of IR light through hole 208. The IR light hits the object and is reflected, at least partially, back through hole 208 and into IR sensor 256. Using the time-of-flight principle, occupancy sensor 250 is able to measure the distance to the object.

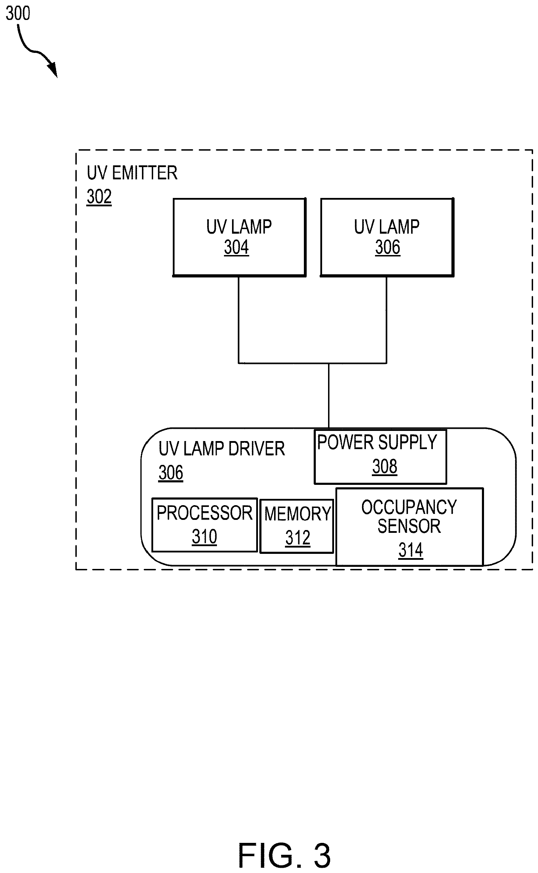

Referring now to FIG. 3, in accordance with various embodiments, a system architecture 300 for a UV emitter 302 is illustrated. UV emitter 302 may be an example of UV emitter 112 discussed above with respect to FIG. 1 or UV emitter 200 discussed above with respect to FIGS. 2A and 2B. UV emitter 302 includes a first UV lamp 304, a second UV lamp 306, and a UV lamp driver 306. First and second UV lamps 304, 306 may be examples of first and second UV light sources 204, 206 described above with respect to FIGS. 2A and 2B.

UV lamp driver 306 includes a power supply 308, a processor 310, a memory 312, and an occupancy sensor 314. Occupancy sensor 314 may be an example of occupancy sensor 250 described above with respect to FIGS. 2A and 2B. In various embodiments, occupancy sensor 314 may be an ultrasonic sensor. Power supply 308 supplies the power used by first and second UV lamps 304, 306. In various embodiments, power supply 308 may be connected to an A/C power source. In various embodiments, power supply 308 may include batteries. In various embodiments, power supply 308 may be connected to the DC power supply of an aircraft.

Processor 310 may comprise one or more processors configured to implement various logical operations in response to execution of instructions, for example, instructions stored on a non-transitory, tangible, computer-readable medium. The one or more processors can be a general purpose processor, a microprocessor, a microcontroller, a digital signal processor (DSP), an application specific integrated circuit (ASIC), a field programmable gate array (FPGA) or other programmable logic device, discrete or transistor logic, discrete hardware components, or any combination thereof. In various embodiments, processor 310 and memory 312 may be external to UV lamp driver 306, and even UV emitter 302, so that processor 310 and memory 312 may control more than one UV emitter 302.

In various embodiments, processor 310 may thus be configured to implement various logical operations in response to execution of instructions. For example, instructions may be stored on a non-transitory, tangible computer-readable medium. In various embodiments, memory 312 may be the non-transitory, tangible computer-readable medium. The term "non-transitory" is to be understood to remove only propagating transitory signals per se from the claim scope and does not relinquish rights to all standard computer-readable media that are not only propagating transitory signals per se.

Processor 310 periodically activates UV emitter 300 to disinfect aircraft lavatory 100. Processor 310 is triggered to activate UV emitter 300, and more specifically, first and second UV lamps 304, 306. Processor 310 may be triggered by a timer, a door switch, occupancy sensor 314, or a button, among others. In various embodiments, the timer may trigger processor 310 to perform a disinfecting cycle every about 5 minutes to about 30 minutes, and more specifically, about 10 minutes to about 20 minutes. In various embodiments, the door switch may trigger processor 310 to perform a disinfecting cycle in response to a passenger leaving aircraft lavatory 100 (i.e., unoccupied). In various embodiments, a crew member or maintenance person may press a button to trigger processor 310 to perform a disinfecting cycle.

Regardless of how processor 310 is triggered, processor 310 turns on UV emitter 300, waits for a period of time, and then turns off UV emitter 300. The period of time, also called cycle time, is dependent on several factors. For example, a target dose may be identified based on a desired reduction of germs on a surface, such as that described above with respect to the SARS-CoV-2 virus. As another example, the distance from the UV emitter 302 to the surface (e.g., toilet 102, floor 110, counter 108) affects the duration of the disinfection cycle, or in other words, how long it takes to reach the target dose. Furthermore, as previously mentioned, the wavelength of UV light used and the power output of the UV light affects the duration of the disinfection cycle. In various embodiments, a target dose may be about 0.5 mJ/cm² to about 20 mJ/cm², or more specifically, about 1 mJ/cm² to about 5 mJ/cm². The time to reach the target dose may be decreased by increasing the strength of the UV emitter 302 and/or by decreasing the distance between UV emitter 302 and the surface.

Once triggered, processor 310 runs and manages a disinfection cycle. Each disinfection cycle begins with a processor 310 measuring the distance between UV emitter 300 and a target surface (e.g., toilet 102, floor 110, counter 108, etc.). In various embodiments, processor 310 may receive distance data from occupancy sensor 314. Beginning each disinfection cycle with a distance measurement allows UV emitter 300 to be placed in any number of locations without first calibrating UV emitter 300 to each location. This reduces time, energy, and expense involved in using UV emitter 300. Processor 310 uses the measured distance to determine the disinfection cycle time to use to reach the target dose. Processor 310 then activates first and second UV lamps 304, 306.

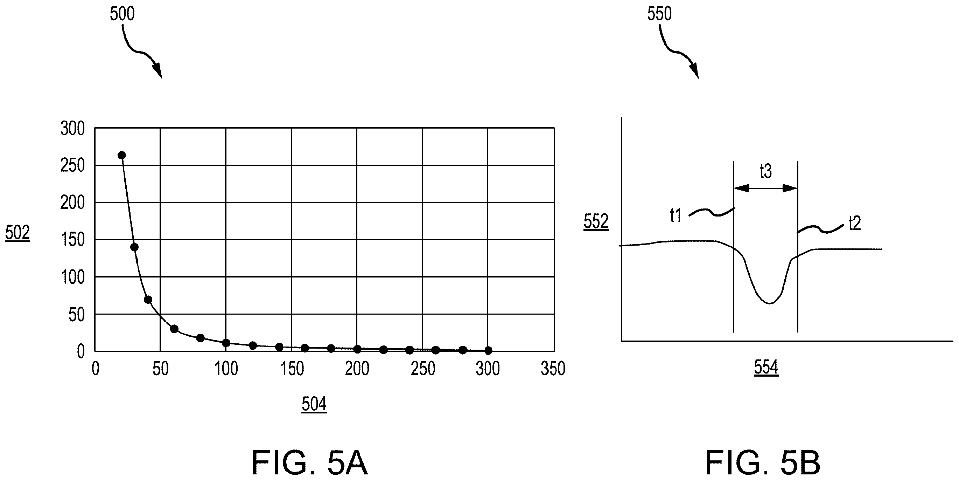

The measured distance affects the duration of the disinfection cycle. Referring to FIG. 5A, a graph 500 illustrates the UV dose 502 measured as µW/cm² as a function of distance 504 measured in cm. As seen, doubling the distance from UV emitter 300 quadruples the duration of exposure to reach a desired dose. For example, at a distance of 60 cm a 1 mJ/cm² dose is reached after 30 seconds. However, at a distance of 120 cm it takes 120 seconds to reach the same 1 mJ/cm² dose.

During each disinfection cycle, processor 310 records a distance to the target surface periodically. In various embodiments, processor 310 records the distance to the target surface every about 0.01 seconds to about 20 seconds, and more specifically, every about 1 second to about 5 seconds. Processor 310 further records the elapsed time during each disinfection cycle. The distance to the target surface is checked periodically in order to detect movement within the disinfection zone of UV emitter 300. Over exposure to UV light can cause problems for fixtures and humans within the disinfection zone. For example, overexposure to UV light can cause discoloration of various surfaces and degradation of the structural integrity of the surface. For humans, the problems are typically discomfort in the presence of UV emitter 300 as safe exposure limits for humans are generally much higher than the target dose of aircraft lavatory 100. For example, safe UV exposure limits for humans are typically about 20 mJ/cm² to about 200 mJ/cm².

Processor 310 periodically calculates an accumulated dose of UV light received on the target surface based in part on the measured distance and the elapsed time. In various embodiments, processor 310 may calculate the accumulated dose after each distance measurement. This allows processor 310 to adjust total duration of the disinfection cycle in response to any change in measured distance. This ensures that the correct dose is received regardless of distance change. In various embodiments, processor 310 may include a time buffer in the calculations to remove anomalous distance changes. For example, when the target surface measures a first distance from UV emitter 302 repeatedly and then one measurement is different. As another example, a person, animal, or object may pass under UV emitter 302 momentarily. In such examples the target dose is not adjusted. This is illustrated in FIG. 5B by graph 550. Graph 550 plots a line 556 as the distance from UV emitter 302 as 502 with respect to the time of exposure as 554. As can be seen, line 556 decreases between a time t1 and a time t2, indicating that the distance from UV emitter 302 decreased between time t1 and time t2. The duration of the decrease is time t3. Processor 310 may choose to discard the time between time t1 and time t2 from dose calculations if time t3 is below a threshold. In various embodiments, the threshold may be about 0.1 second to about 30 seconds, and more specifically, about 1 second to about 5 seconds. A change in distance occurring for less than time t3 may be considered an anomaly and discarded, that is, not used in the calculation for the accumulated dose.

Processor 310 deactivates first and second UV lamps 304, 306 in response to reaching the target dose. In various embodiments, processor 310 may deactivate first and second UV lamps 304, 306 a short time before reaching the target dose. In various embodiments, processor 310 may deactivate first and second UV lamps 304, 306 a short time after reaching the target dose. In various embodiments, processor 310 may include an analog timer (e.g., a watchdog timer) that causes processor 310 to deactivate first and second UV lamps 304, 306 after a timeout time that exceeds the target dose time. In various embodiments, the timeout time may be about 2 to about 5 times longer than the target dose time. The analog timer provides a maximum duration of UV light activation. This provides a safety mechanism to reduce thermal stress on first and second UV lamps 304, 306 and prevent discoloration, among others. Additionally, there is little to no disinfection benefit to exceeding the target dose time. The analog timer (e.g., watchdog timer) may be reached in situations where occupancy sensor 314 fails or provides an inaccurate result such as an error to read a distance. Such a error may result in a long disinfection cycle time because the target dose time was incorrectly calculated.

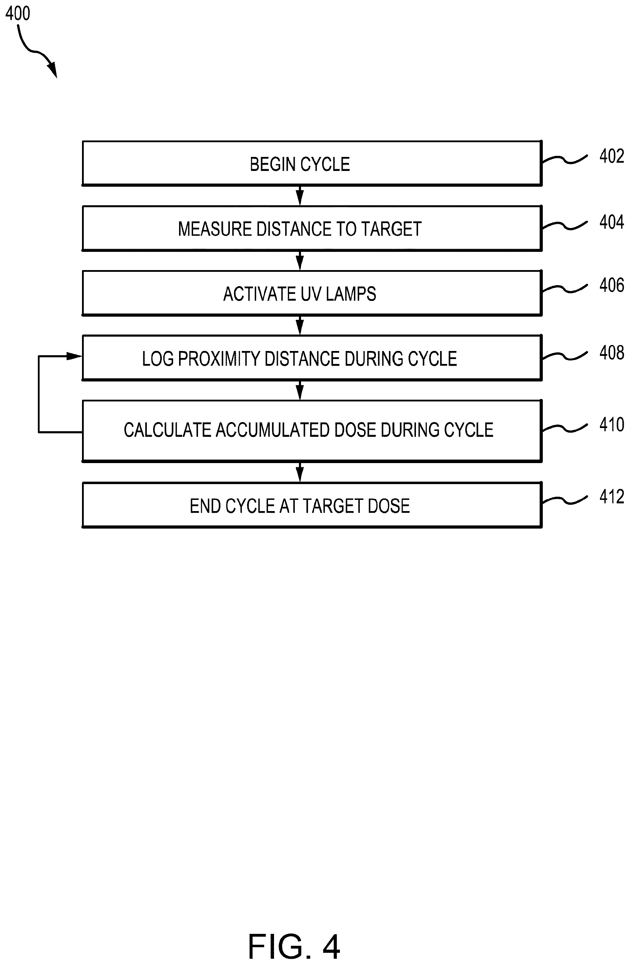

Referring now to FIG. 4, in accordance with various embodiments, a method 400 for controlling a UV emitter is illustrated. Method 400 may be performed by processor 310 described above with respect to FIG. 4. UV emitter may be UV emitter 112 described above with respect to FIG. 1. In various embodiments, UV emitter may be UV emitter 300 described above with respect to FIG. 3. Method 400 describes several steps in a disinfection cycle as described above with respect to FIG. 3.

At block 402, processor 310 begins the disinfection cycle.

At block 404, processor 310 measures the distance to the target. In various embodiments, processor 310 requests a distance reading from occupancy sensor 314. In various embodiments, processor 310 may be part of a controller that includes a proximity sensor.

At block 406, processor 310 activates the UV lamps of the UV emitter 300. In various embodiments, UV emitter 300 may have more than one UV lamp. Processor 310 may activate one or more lamps in response to beginning the disinfection cycle.

At block 408, processor 310 records the distance to the surface during the disinfection cycle. Processor 310 may record the distance once every about 0.01 seconds to about 10 seconds, and more specifically, every about 1 second to about 5 seconds. Periodically measuring the distance to the surface during the disinfection cycle ensures that nothing has been moved in between UV emitter 300 and the surface. This protects the lamps of UV emitter 300 from overheating and the surface from discoloration and structural degradation.

At block 410, processor 310 calculates the accumulated dose during the disinfection cycle. The accumulated dose is based at least in part on the power output of the UV lamps (e.g., first and second UV lamps 304, 306), the distance to the surface, and the elapsed time. Processor 310 may repeat block 408 and 410 throughout the disinfection cycle.

At block 412, processor 310 ends the disinfection cycle and deactivates the UV lamps 304, 306 in response to the target dose being reached. In various embodiments, processor 310 may record a total lamp time used to predict lamp failure. Processor 310 may provide an indication of an upcoming lamp failure by, for example, blinking the UV lamps at the beginning or the end of a disinfection cycle. Upcoming lamp failure may be defined as the UV lamps having less than about 10% of expected life remaining, measured in minutes or hours of use.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 10%, within 5%, within 1%, within 0.1%, or within 0.01% of a stated value. Additionally, the terms "substantially," "about" or "approximately" as used herein represent an amount close to the stated amount that still performs a desired function or achieves a desired result. For example, the term "substantially," "about" or "approximately" may refer to an amount that is within 10% of, within 5% of, within 1% of, within 0.1% of, and within 0.01% of a stated amount or value.

Finally, it should be understood that any of the above described concepts can be used alone or in combination with any or all of the other above described concepts. Although various embodiments have been disclosed and described, one of ordinary skill in this art would recognize that certain modifications would come within the scope of the invention that fall within the scope of the claims.

## Claims

1. A method of using an ultraviolet, UV, light emitter, comprising:
measuring, by a processor (310), a first distance from the UV light emitter (302) to a target surface;
activating, by the processor, the UV light emitter;
recording, by the processor, a second distance from the UV light emitter to the target surface;
calculating, by the processor, an accumulated dose of UV light on the target surface based at least in part on the second distance; and
deactivating, by the processor, the UV light emitter in response to the accumulated dose reaching a target dose.

2. The method of using the UV light emitter of claim 1, wherein the recording the second distance occurs once every second.

3. The method of using the UV light emitter of claim 1 or 2, further comprising:
recording, by the processor, an accumulated time since activating the UV light emitter; and
calculating, by the processor, the accumulated dose of UV light based at least in part on the accumulated time and the recorded second distance.

4. The method of using the UV light emitter of any preceding claim, wherein the calculating the accumulated dose includes integrating the second distance by an energy output of the UV light emitter over an accumulated time period.

5. The method of using the UV light emitter of any preceding claim, further comprising:
measuring, by the processor, a third distance from the UV light emitter; and
detecting, by the processor, a movement in response to the third distance being different than the second distance.

6. The method of using the UV light emitter of claim 5, further comprising:
changing, by the processor, the target dose to a lower dose in response to the third distance being less than the second distance.

7. The method of using the UV light emitter of claim 5 or 6, further comprising:
measuring, by the processor, a fourth distance from the UV light emitter; and
maintaining, by the processor, the target dose in response to the fourth distance being similar to the second distance, and optionally further comprising:
measuring, by the processor, a time between the second distance measurement and the fourth distance measurement; and
determining, by the processor, that the time is below a threshold.

8. The method of using the UV light emitter of any preceding claim, further comprising:
repeating, by the processor, the recording and the calculating until the target dose reached.

9. The method of using the UV light emitter of any preceding claim, wherein the measuring the first distance is performed in response to a trigger.

10. A system for using an ultraviolet, UV, light emitter in an aircraft lavatory, comprising:
a UV light source (304, 306);
an occupancy sensor (314);
a UV light source driver (306) including:
a power source (308) configured to power the UV light source;
a processor (310); and
a memory (312) coupled to the processor, the memory comprising instructions when executed by the processor cause the processor:
measure a first distance from the occupancy sensor to a target surface;
activate the UV light source;
record a second distance from the occupancy sensor to the target surface;
calculate an accumulated dose of UV light on the target surface based at least in part on the second distance; and
deactivate the UV light source in response to the accumulated dose reaching a target dose.

11. The system for using the ultraviolet, UV, light emitter in an aircraft lavatory of claim 10, wherein the instructions when executed by the processor further cause the processor to:
record the second distance each time period; and
calculate the accumulated dose each time period; and/or
wherein the instructions when executed by the processor further cause the processor to:
record an accumulated time since activating the UV light source; and
calculate the accumulated dose based at least in part on the accumulated time and the recorded second distance.

12. The system for using the ultraviolet, UV, light emitter in an aircraft lavatory of claim 10 or 11, wherein the calculating the accumulated dose includes integrating the second distance by an energy output of the UV light emitter over an accumulated time period.

13. The system for using the ultraviolet, UV, light emitter in an aircraft lavatory of claim 10, 11 or 12, wherein the instructions when executed by the processor further cause the processor to:
measure a third distance from the occupancy sensor to the target surface; and
detect a movement in response to the third distance being different than the second distance; and optionally wherein the instructions when executed by the processor further cause the processor to:
change the target dose to a lower dose in response to the third distance being less than the second distance; and/or wherein the instructions when executed by the processor further cause the processor to:
measure a fourth distance from the occupancy sensor; and
determine to maintain the target dose in response to the fourth distance being similar to the second distance; and/or
wherein the instructions when executed by the processor further cause the processor to:
measure a time between the second distance measurement and the fourth distance measurement; and
determine that the time is below a threshold before determining to maintain the target dose.

14. The system for using the ultraviolet, UV, light emitter in an aircraft lavatory of any of claims 10 to 13, wherein the instructions when executed by the processor further cause the processor to:
begin the measuring the first distance in response to a trigger.

15. The system for using the ultraviolet, UV, light emitter in an aircraft lavatory of any of claims 10 to 14, wherein the instructions when executed by the processor further cause the processor to:
repeat the recording and the calculating until the target dose is reached.
